(19) Europäisches Patentamt
European Patent Office
Office européen des brevets

(11) EP 2 072 002 A1

(12) EUROPEAN PATENT APPLICATION

(43) Date of publication:
24.06.2009 Bulletin 2009/26

(51) Int Cl.:
*A61B 1/005* (2006.01)

(21) Application number: 07150257.9

(22) Date of filing: 20.12.2007

(84) Designated Contracting States:
AT BE BG CH CY CZ DE DK EE ES FI FR GB GR
HU IE IS IT LI LT LU LV MC MT NL PL PT RO SE
SI SK TR
Designated Extension States:
AL BA HR MK RS

(71) Applicant: Nederlandse Centrale Organisatie Voor
Toegepast
Natuurwetenschappelijk Onderzoek TNO
2628 VK Delft (NL)

(72) Inventors:
• Heijmans, Jeroen Antonius Cecillia
5261 EL Vught (NL)
• Verbaan, Cornelis Abraham Marinus
4225 PM Noordeloos (NL)

(74) Representative: Hatzmann, Martin et al
Vereenigde
Johan de Wittlaan 7
2517 JR Den Haag (NL)

(54) **A bendable structure and a method for bending a structure**

(57) The invention relates to a bendable structure 40 comprising a body 41 conceived to be bent; an actuator 42, 43, 44 for inducing a bending force in the body, wherein the actuator comprises a wire at least partially manufactured from a shape memory alloy (SMA) material, said wire being pre-deformed and being arranged in contact with a portion of the body for forming a bridge structure The bendable structure 40 may relate to a bendable catheter or an endoscope.

Fig.3

EP 2 072 002 A1

**Description**

FIELD OF THE INVENTION

The invention relates to a bendable structure. In particular, the invention relates to a bendable medical instrument, like a catheter or an endoscope. The invention further relates to a method of bending a structure.

BACKGROUND OF THE INVENTION

An embodiment of a bendable structure is known from Ki-Tae Park et al "An active catheter with integrated circuit for communication and control". In the known bendable structure bending functionality is enabled by providing a tubular body of the catheter with coils fabricated from a shape memory alloy (SMA). SMA materials are known as such in the art and relate to a class of materials which may be deformed in a controlled way, for example by application of a current pulse. Details on SMA materials may be found in M. Langelaar and F. van Keulen "Modelling of a shape memory alloy active catheter".

The known bendable structure comprises a plurality of segments manufactured from SMA coils, said segments being consecutively interconnected by links. The SMA coils are pre-deformed having 3% deformation strain. When the SMA actuator is heated above its phase transition temperature by electric current and starts recovering its original shape, the active catheter bends in the direction of the heated SMA actuator. In order to implement bending of the known bendable structure a total of three SMA actuator wires are provided within a body conceived to be bent, these three actuator wires are arranged at vertices of an imaginary triangle fitted into a cross-section of the body.

It is a disadvantage of the known bendable body that bending with poor accuracy can be achieved. Next, the known bendable structure has limited bending angles. Finally, the known bendable structure cannot be adequately miniaturized due required use of at least three SMA actuator wires.

SUMMARY OF THE INVENTION

It is an object of the invention to provide a bendable structure wherein greater bending accuracy can be reached. Additionally, it is a further object of the invention to provide a bendable structure wherein greater bending angles can be enabled. It is a still further object of the invention to provide a bendable structure having substantially diminished power dissipation into surroundings upon actuation.

To this end the bendable structure according to the invention comprises:

- a body conceived to be bent;
- an actuator for inducing a bending force in the body, wherein the actuator comprises a wire at least partially manufactured from a shape memory alloy (SMA) material, said wire being pre-deformed and being arranged in contact with a portion of the body for forming a bridge structure.

The invention is based on the insight that the SMA wire when pre-deformed relaxes during activation and in course of relaxation the bending force can be transferred to another side of the body across the bridge structure. There are two principal embodiments envisaged for the bridge structure.

First, the body may comprise an elastic hinge, whereas the bridge structure may be formed by the elastic hinge connected to the wire. In a particular embodiment, when the elastic hinge is manufactured from a SMA material, the bending force transferred from the SMA wire can be stored in an opposite arm of the elastic hinge. This feature is based on the insight that the hinge can be implemented as a spring-like element, in particular when for a material of the flexible hinge a material having pseudo-elastic properties is selected. Advantageously, when such material exhibits austenitic properties at room temperature, it can be brought in the martensitic state by inducing deformation. When a force causing the deformation is relieved, the material will return in it original shape. In general such materials are similar to rubber when transiting from austenitic to martensitic state. It is appreciated that during transition from the martensitic to austenitic phase substantially great forces are relieved. Use of pseudo elastic material is advantageous for reasons of low bending stiffness and large allowable strain (bending angle). Secondly, by choosing a unidirectional SMA for the hinge material, both the low bending stiffness, large allowable strain, and the energy that can be stored are advantageous.

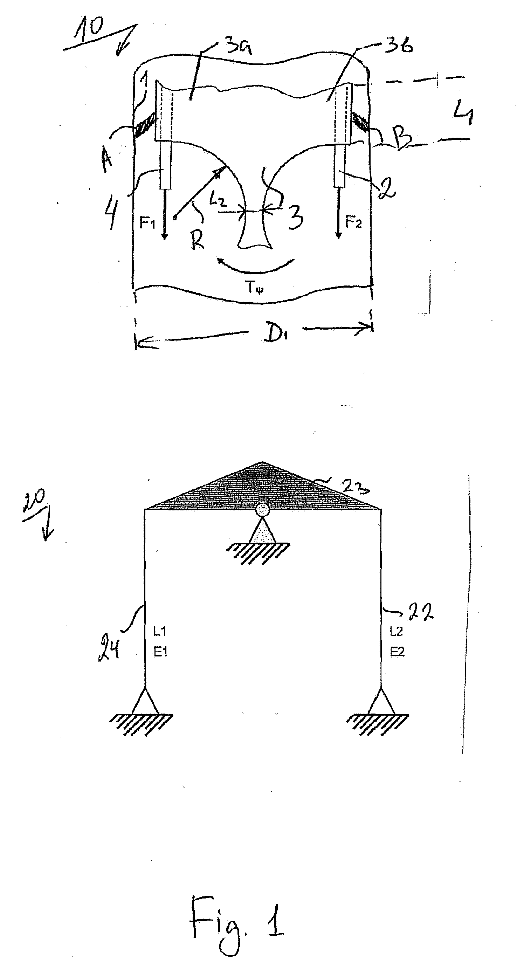

Details on operation of the bridge structure are explained with reference to Figure 1. When the opposite side of the SMA hinge is actuated, the force is transferred back to the SMA wire. In this way bending force induced in the bendable structure is being transferred from one side of the bridge to another side of the bridge in accurate way. In addition, this embodiment uses only one actuation SMA wire and is, therefore, preferable in situations when the bendable structure has to meet miniaturization requirements, for example for minimal invasive surgery, such as miniature endoscopes. Alternatively, a miniaturized device may be used for industrial applications, for example for purposes of engine or gear inspection.

Secondly, it is possible that the bridge structure comprises a further deformable SMA wire joined by a rigid hinge to the pre-deformed wire.

In this case the bridge structure may represent a substantially rigid hinge connected to two deformable SMA wires acting as it arms. It will be appreciated that the term 'rigid' relates to a material of the hinge being substantially non-deformable. Preferably, the hinge material has a Young modulus of more than IGPa at T=20°C.

The hinge interconnecting the pre-deformed wire and the further wire forms a mechanical bridge for transferring a bending momentum from one side of the hinge to another thereby causing the body of the bendable structure to bend. It will be appreciated that the bridge has a pre-stored mechanical energy due to the fact that the first SMA wire is pre-deformed. In this way, when the electrical current pulse of a suitable duration is applied to the pre-deformed first wire and the material of the wire is heated above a phase transition temperature, the SMA wire create a force on the further deformable SMA wire. In response, the second deformable SMA wire will be elongated until equilibrium of forces across the bridge is reached. In this way the mechanical energy is transferred to the second wire and is stored there. When the second SMA wire is being heated above its phase transition temperature by means of electrical current or otherwise, the second wire will be shortening in response and will thereby create a force on the first (now relaxed) SMA wire. As a result the first SMA wire will be elongated until equilibrium of forces along the bridge is reached. Now, the mechanical energy is being transferred again across the bridge and being stored in the first wire. By repeating alternating heating the first wire and the second wire the controlled bending of the bendable structure is achieved. This phenomenon is explained in more detail with reference to Figure 1.

The bendable structure according to the invention has the following advantages. First, due to provision of the bridge structure accurate bending actuation is reached due to good controllability of the SMA phase transition. In addition, when for the SMA material a uni-directional material is used a small amount of activation energy is required to bend the bendable structure. Uni-directional memory material relates to a material which can revert to its original shape, which is defined a-priori. Secondly, the activation energy is only applied for a short duration, for example a current pulse of 0.1 - 10 s duration, preferably 5s duration can be used to achieve necessary bending angle. This is advantageous with respect to the prior art when the activation pulse has to be applied continuously leading to a great energy dissipation in surroundings. This may be not acceptable for medical applications wherein heating of surrounding tissue is not allowable.

Preferably, the SMA wire is pre-deformed by elongation by 4-8% of its initial length. This means that for reaching envisaged bending radii of about 10mm for a 1mm diameter, at least 4-8% elongation of the SMA wire is advantageous. It will be appreciated that it is also possible that both SMA wires are pre-deformed for a portion of 4 - 8% range.

In an embodiment of the bendable structure according to the invention the uni-directional shape memory alloy is selected from a group of materials comprising: NiTi, CuZnAl or CuAlNi.

As has been mentioned earlier, shape memory property is a functionality of a material to transit to its original shape from a deformed condition after it is heated. This phenomenon is based on a phase transition in the crystal structure during cooling of a rigid, high temperature state (austenitic) to a less rigid lower temperature state (martensitic) and vice versa during heating. This phase transformation is reversible and, therefore, can easily be used for actuation purposes. It will be appreciated that there is another transition possible for the shape memory alloys, namely a transition between the austenitic state and the so-called R-phase. The R-phase corresponds to rhombohedral crystal orientation. It is found that it is preferable to use the transformation between the austenitic phase and the martensitic phase in case smaller bending radii are required. For larger bending radii it is advantageous to use the transformation between the austenitic phase to the R-phase because such transition is linear as a function of temperature and has little hysteresis, which is advantageous for simplifying control of the bending.

In a further embodiment of the bendable structure according to the invention the body has a low bending stiffness, which may be implemented when for the material of the hinge a SMA material is selected, in particular a material having pseudo-elastic properties.

This is advantageous, as is this case the body demonstrates substantially no resistance to bending and substantially no backwards recoiling. This further improves bending accuracy.

In a still further embodiment of the bendable structure according to the invention, it further comprises control means for inducing a transition of the shape memory alloy from martensitic phase to austenitic phase or from austenitic phase to R-phase.

Preferably, the control means is arranged to apply a pulse of electrical current of suitable duration and/or amplitude for enabling a transition of the shape memory alloy from martensitic phase to austenitic phase or from austenitic phase to R-phase.

The control means may be advantageously arranged to use a pre-calibrated dependency between a desired bending radius of the bendable structure and the duration or amplitude of a current pulse conceived to be applied to the SMA wires for enabling such bending. This feature further improves the bending accuracy. This short term activation (0.1s - 10 s, preferably about 5s) has an advantage of reduced power dissipation on the bendable structure, which is preferable for medical applications due to strict limitation on local heating of tissue.

In a further embodiment of the bendable structure according to the invention the elastic hinge comprises a plurality of interconnected hinge elements extending in a longitudinal direction of the body.

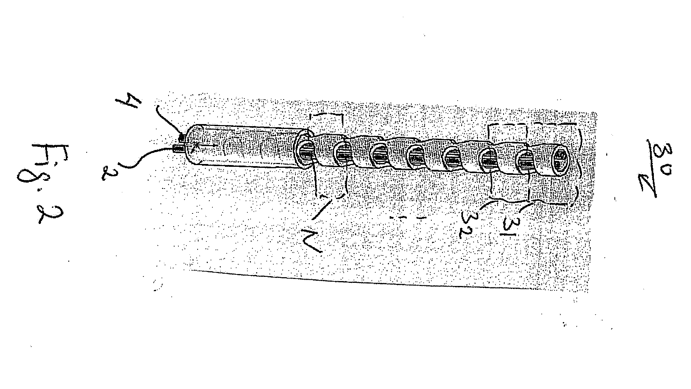

This technical feature is based on the insight that by providing a continuous structure having a plurality of interconnected bridge structures the accuracy of the bending is improved. In particular, when the elastic hinge comprises a plurality of interconnected hinge elements having substantially tubular cross-section and provided with recesses, controlled bending with preservation of the shape of the cross-section is achieved. This is of particular advantage for medical applications. More details on this embodiment will be presented with reference to Figure 2.

A method for bending a structure according to the invention comprises the steps of:

- selecting for the structure a body arranged with an actuator in contact with the body for transforming a bending force to the body, said actuator comprising a wire at least partially manufactured from a shape memory alloy (SMA) material, said wire being pre-deformed and being arranged in contact with a portion of the body for forming a bridge structure;
- providing actuation signals to the wire thereby bending the structure.

In a particular embodiment of the method according to the invention, wherein the bridge structure comprises a further deformable SMA wire conceived to interact with the pre-deformed wire, the method comprises the steps of alternatively actuating the wire and the further wire.

These and other aspects of the invention will be further discussed with reference to drawings, wherein like reference signs represent like elements. It will be appreciated that the drawings are used for illustrative purposes only and may not be construed for limiting the scope of invention of appended claims.

BRIEF DESCRIPTION OF THE DRAWINGS

Figure 1 presents a schematic view of a bridge principle used in the bendable structure according to the invention.
Figure 2 presents a schematic view of a hinge comprising a plurality of hinge elements.
Figure 3 presents a schematic view of a medical device comprising the bendable structure according to the invention.

DETAILED DESCRIPTION OF THE DRAWINGS

Figure 1 presents a schematic view of a bridge principle used in the bendable structure according to the invention. The bendable structure 10 comprises a body 1, preferably a tubular body being manufactured from an elastic material. The body is provided with actuator comprising a first SMA wire 4, a second SMA wire 2 and a substantially rigid hinge 3 arranged to mechanically interconnect the first wire 2 and the second wire 4 for obtaining a bridge. An equivalent mechanical scheme is presented by element 20, illustrating the first wire 24, the second wire 22 and the bridging element 23. The first wire 4 is preferably pre-deformed by at least 4 - 8 % of its length. It is possible that the wire is elongated by at least 4 - 8 % of its length. Due to the fact that the first wire is at least partially manufactured from a suitable SMA material, upon an effect the wire is heated above its phase transition temperature, for example by application of a current pulse of a suitable duration and amplitude, the first wire will recover its normal shape, i.e. shorten thereby pulling the portion 3a of the hinge to the left. Due to the fact that the hinge 3 is manufactured from a substantially rigid material, the force applied by the first wire 4 to the portion 3a will be transferred substantially without loss to the portion 3b of the hinge 3. As a result the wire 2 will be elongated until equilibrium of forces is reached. As a result the second wire 2 will store mechanical energy. When the second wire is heated above its phase transition temperature, the second wire will resume its original length pulling the portion 3b to the right. As a consequence the first wire 4 will be elongated. Thus, by alternating application of current pulses of suitable amplitude and duration, controlled bending of the body is reached. It will be appreciated that in order to bend the body in one direction at least two wires 2, 4 are required. The hinge is connected to the body 1, which is schematically indicated by arms A, B. It will be appreciated that in practice instead of arms other technical means may be used, like adhesive or the like. Alternatively, the actuator comprising the hinge and the wires may be tightly fitted in the interior of the body 1 so that bending of the actuator is substantially transferred into bending of the body 1. It will be appreciated that for enabling controlled bending of the structure 10 in other directions additional wires may be required.

The hinge element 3 may be preferably manufactured from a tubular structure by means of laser ablation for producing voids having radius R. The absolute dimensions of the hinge 3 and the radius R depend on application. For example, for application in a bendable endoscope, it is possible that the outer dimension of the body is 0.7 mm, the inner dimension of the body is 0.5 mm L1 is about 0.5 mm, L2 is about 0.1 mm and radius R is about 0.5 mm. In order to determine feasible bending angles for this structure, the following equations are to be considered:

$$M = F * L ,$$

wherein

M represents moment applied to a point;
F represents force acting on a point;
L - represents arm length.

$$F = A * E * \varepsilon \ (\text{Hook's law})$$

During actuation of the perforated hinge as shown in Figure 2, the hinge will rotate in direction of the wire which is being actuated. Although the explanations are given with respect to the wires which are elongated and which shorten under application of the actuation pulse, it is also possible that the wire is shortened a-priori and elongates during application of the actuation pulse.

The rotation of the hinge proceeds until equilibrium of forces across the bridge (see Figure 1 element 20) is reached. In this state a sum of momenta in the bendable structure is zero, which is given by:

$$\sum M = 0$$

By filling in data pertaining to the geometry of the hinge, one obtains:

$$F1 * l1 - F2 * l2 - T\psi = 0$$

When the above equation is resolved with respect to the angle Ψ, one obtains that the forces equilibrium is maintained for the angle Ψ of 7,2 degrees. By providing a suitable plurality of the hinge elements 31,32, ..., N, as shown in Figure 2 a suitable bending angle is obtained. For example, for 30 elements forming the hinge 30 the bending angle of 216 degrees is reached. It is noted that when the body comprises an elastic hinge and the bridge structure is formed by the elastic hinge connected to the wire, in particular, when the elastic hinge is manufactured from a uni-directional SMA material, it is sufficient to use a sole actuating SMA per desired bending direction, the elastic hinge acting as the second SMA wire.

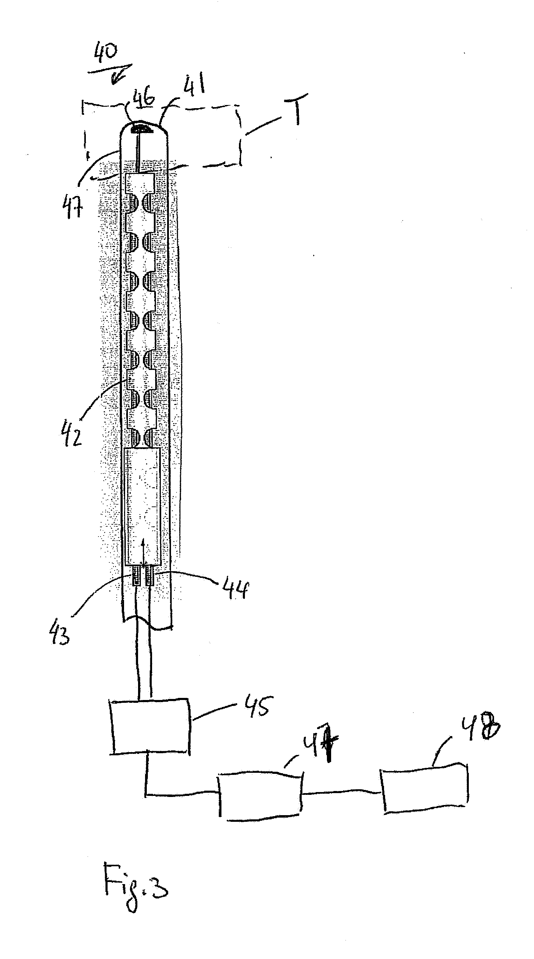

Figure 3 presents a schematic view of a medical device comprising the bendable structure according to the invention. The bendable structure 40 may relate to a bendable catheter or an endoscope. The catheter may be suitable to be maneuvered in a body's conduit, for example in a blood vessel or in a urinary tract. Below, example of the bendable structure implemented in a miniature endoscope will be given. It will be appreciated that a skilled artisan can employ the same teaching in application to catheters or any other suitable equipment requiring remote controlled bending.

Endoscope 40 may comprise an outer tubular body 41, whereto a flexible hinge 42 arranged with SMA wires 43, 44 is attached. It is also possible that the endoscope 40 comprises a suitable lumen occupying only a portion of the internal volume, the hinge 42 together with the wires 43, 44, being arranged in the lumen. Preferably, the tubular body 41 has a diameter in the range of 0.5 - 10 mm, preferably in the range of 0.5 - 2 mm.

The SMA wires are actuated by means of application of a pulse of electrical current for heating the SMA wire above its phase transition temperature. It is noted that either a transition of a suitable SMA material from martensitic phase to austenitic phase or from austenitic phase to R-phase is envisaged. In order to actuate the wires 43, 44 the endoscope 45 comprises a control unit, which is arranged to deliver the current pulse with required characteristics to the first wire 43 or to the second wire 45.

In a medical domain, it is advantageous that a protrusion of the endoscope 40 in the human or animal body is being monitored in real-time. Preferably, such monitoring is arranged to prove a three-dimensional tracking of a position of the endoscope. Advantageously, the control unit 45 is arranged to receive the positional information on the endoscope together with imaging data on the area wherein the endoscope, in particular its tip portion T is dwelling. The bending angle can also be monitored using a strain sensor or optical fibre.

In accordance with the imaging data the control unit 45 or a suitable data analysis unit 47 arranged in communication with a suitable imaging unit 48 calculates the angle with which the tip portion T of the endoscope is to be bent prior to further insertion. It will be appreciated that the same procedure can be followed when an inspection endoscopy is performed, for example when the endoscope is positioned within an organ, or a cavity and is moved around to enable a wide view for the optical means 46. The optical means 46 is arranged in connection with an external device (not shown) for further processing of the data. Such connection is preferably implemented using optical fibre.

Preferably, the SMA wires 43, 44 are arranged with a flattened cross-section. This has an advantage that less space is occupied by the wires 43, 44. This feature is of particular advantage for catheters conceived to be used in cardiac arteries, because miniaturization of cardiac devices plays a crucial role with respect to induced ischemia.

It will be appreciated that while specific embodiments of the invention have been described above, that the invention may be practiced otherwise than as described. In addition, isolated features discussed with reference to different figures may be combined. Although the bendable structure is explained with reference to endoscope other applications are contemplated, including other optical devices, for example for industrial application, catheters, or the like.

## Claims

1. A bendable structure comprising:

   - a body conceived to be bent;
   - an actuator for inducing a bending force in the body, wherein the actuator comprises a wire at least partially manufactured from a shape memory alloy (SMA) material, said wire being pre-deformed and being arranged in contact with a portion of the body for forming a bridge structure.

2. A bendable structure according to claim 1, wherein the body comprises an elastic hinge, the bridge structure being formed by the elastic hinge connected to the wire.

3. A bendable structure according to claim 2, wherein the body comprises a plurality of interconnected hinges extending in a longitudinal direction of the body.

4. A bendable structure according to claim 2 or 3, wherein the body comprises SMA material.

5. A bendable structure according to any preceding claim, wherein for the shape memory alloy a uni-directional shape memory alloy is used.

6. A bendable structure according to claim 1, wherein the bridge structure comprises a further deformable SMA wire joined by a rigid hinge to the pre-deformed wire.

7. A bendable structure according to claim 5 or 6, wherein the uni-directional shape memory alloy is selected from a group of materials comprising: NiTi, CuZnAl or CuAlNi.

8. A bendable structure according to any preceding claim, wherein the body has a low bending stiffness.

9. A bendable structure according to any preceding claim, further comprising control means for inducing a transition of the shape memory alloy from martensitic phase to austenitic phase or from austenitic phase to R-phase.

10. A bendable structure according to claim 9, wherein the control means is arranged for controlling of the shape memory alloy by application of a current pulse of a short duration.

11. A bendable structure according to any preceding claims, wherein the bridge structure is formed as a carrier of the body.

12. A bendable structure according to any preceding claim, wherein the body is tubular, having a diameter in the range of 0.5 - 10 mm, preferably in the range of 0.5 - 2 mm.

13. A bendable structure according to any preceding claim, wherein the body forms part of an optical device.

14. A bendable structure according to claim 13, wherein the optical device is an endoscope.

15. A bendable structure according to any preceding claim 1 - 12, wherein the body forms part of a catheter.

16. A bendable structure according to any preceding claim, wherein the wire and the further wire have a flattened cross-section.

17. A bendable structure according to any preceding claim, wherein the deformable wire is elongated by 4 - 8 % of its initial length.

18. A bendable structure according to any preceding claim 6 - 17, wherein the wire and the further wire are pre-deformed.

19. A method for bending a structure comprising the steps of:

- selecting for the structure a body arranged with an actuator in contact with the body for transforming a bending force to the body, said actuator comprising a wire at least partially manufactured from a shape memory alloy (SMA) material, said wire being pre-deformed and being arranged in contact with a portion of the body for forming a bridge structure;
- providing actuation signals to the wire thereby bending the structure.

20. A method according to claim 19, wherein the bridge structure comprises a further deformable SMA wire conceived to interact with the pre-deformed wire, the method comprising the steps of alternatively actuating the wire and the further wire.

Fig. 1

Fig. 2

Fig. 3

European Patent Office

# EUROPEAN SEARCH REPORT

Application Number

EP 07 15 0257

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 679 216 A (TAKAYAMA SHUICHI [JP] ET AL) 21 October 1997 (1997-10-21) | 1,5,6,8, 10,11, 13-15, 19,20 | INV. A61B1/005 |
| Y | * abstract * <br><br> * column 5, line 30 - column 7, line 28 * <br> ----- | 2,3,7,9, 16-18 | |
| X | US 4 753 223 A (BREMER PAUL W [US]) 28 June 1988 (1988-06-28) <br><br> * the whole document * | 1-4, 6-15,19, 20 | |
| Y | ----- | 16-18 | |
| X | US 5 897 488 A (UEDA YASUHIRO [JP]) 27 April 1999 (1999-04-27) | 1,5-8, 10,11, 13,14, 19,20 | |
| Y | <br> * column 9, line 46 - column 10, line 67 * <br> ----- | 2,3 | |
| X | US 2005/154261 A1 (OHLINE ROBERT M [US] ET AL) 14 July 2005 (2005-07-14) * the whole document * <br> ----- | 1,19 | TECHNICAL FIELDS SEARCHED (IPC) <br> A61B |
| Y | WO 2007/034664 A (OLYMPUS CORP [JP]; KITAGAWA HIDEYA [JP]; ADACHI KATSUTAKA [JP]; SUZUKI) 29 March 2007 (2007-03-29) * abstract; figures 2-4 * <br> ----- | 2,3 | |
| Y | US 5 220 911 A (TAMURA HISAAKI [JP]) 22 June 1993 (1993-06-22) * column 3, lines 45-47 * <br> ----- | 7 | |
| Y | WO 99/60267 A (MAYNARD RONALD S [US]) 25 November 1999 (1999-11-25) * page 23 * <br> ----- | 9 | |

The present search report has been drawn up for all claims

| Place of search | Date of completion of the search | Examiner |
|---|---|---|
| Munich | 15 April 2008 | Rivera Pons, Carlos |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................
& : member of the same patent family, corresponding document

EPO FORM 1503 03.82 (P04C01)

EP 2 072 002 A1

ANNEX TO THE EUROPEAN SEARCH REPORT
ON EUROPEAN PATENT APPLICATION NO.

EP 07 15 0257

This annex lists the patent family members relating to the patent documents cited in the above-mentioned European search report.
The members are as contained in the European Patent Office EDP file on
The European Patent Office is in no way liable for these particulars which are merely given for the purpose of information.

15-04-2008

| Patent document cited in search report | | Publication date | Patent family member(s) | | Publication date |
|---|---|---|---|---|---|
| US 5679216 | A | 21-10-1997 | NONE | | |
| US 4753223 | A | 28-06-1988 | NONE | | |
| US 5897488 | A | 27-04-1999 | NONE | | |
| US 2005154261 | A1 | 14-07-2005 | NONE | | |
| WO 2007034664 | A | 29-03-2007 | NONE | | |
| US 5220911 | A | 22-06-1993 | JP | 4122233 A | 22-04-1992 |
| WO 9960267 | A | 25-11-1999 | AT | 236355 T | 15-04-2003 |
| | | | AU | 3978699 A | 06-12-1999 |
| | | | CA | 2332234 A1 | 25-11-1999 |
| | | | DE | 69906508 D1 | 08-05-2003 |
| | | | DE | 69906508 T2 | 19-02-2004 |
| | | | EP | 1076774 A1 | 21-02-2001 |
| | | | JP | 2002515566 T | 28-05-2002 |
| | | | US | 6447478 B1 | 10-09-2002 |

EPO FORM P0459

For more details about this annex : see Official Journal of the European Patent Office, No. 12/82

11